# EUROPEAN PATENT SPECIFICATION

(11) **EP 2 692 332 B1**
(45) Date of publication and mention of the grant of the patent: **21.08.2019**
(21) Application number: 13169536.3
(22) Date of filing: 28.05.2013
(51) Int. Cl.: A61K 8/36, A61K 8/37, A61Q 19/00, A61K 31/19, A61K 31/194

(54) **Composition for the treatment of callus, corns and psoriasis**
Zusammensetzung zur Behandlung von Callus, Hühneraugen und Psoriasis
Composition pour le traitement de cal, cors et psoriasis

(30) Priority: 29.05.2012 DK 201270286
(43) Date of publication of application: 05.02.2014
(73) Proprietor: Unigroup ApS, 2800 Lyngby (DK)
(72) Inventor: Licht, Flemming, 2720 Vanløse (DK)
(74) Representative: Holme Nielsen, Marianne

(56) References cited:
- EP-A1- 2 460 509
- WO-A1-00/45808
- WO-A1-2011/028110
- WO-A2-2007/113830
- US-A1- 2008 153 757

## Description

The present invention relates to compositions for the treatment of callus, corns, psoriasis, In particular, it concerns compositions for the topical treatment of callus, corns, and psoriasis on mammals, such as humans.

### Technical Background

The article "Salicylic acid in the treatment of corns" by L.M.G. Lang et al., The Foot, Volume 4, Issue 3, September 1994, Pages 145-150, describes the use of 40% w/w salicylic acid corn plasters for removing corns. Corns may be caused by extrinsic and/or intrinsic factors. A common cause is pressure from ill fitting footwear and irritations from irregularities in shoes and hosiery. Intrinsic causes of corns include bony prominences or faulty foot function and structure. The hard corn (heloma durum) is a circumscribed area of hyperkeratotic tissue which, on the dorsum of the foot, often presents over the interphalangeal joint of the toes. Pain from corns may be sharp and severe or dull and discomforting.

The International Patent Application WO 2011/028110 A1 describes compositions for the treatment of skin conditions comprising at least one physiologically acceptable C1-C4 alkyl ester of a carboxylic acid as a carrier, and salicylic acid and/or a physiologically acceptable ester of salt thereof.

The International Patent Application WO 00/45808 describes the use of a preparation comprising formic acid as an active ingredient for the manufacture of a medicament for treating skin warts caused by papilloma virus(es) in a mammal by topical administration on the affected area.

### Summary of the invention

The use of salicylic acid for removing corns may be associated with pain, swelling, heat and throbbing from the treatment site, as well as maceration. Using a stronger acid implies greater risk of harming the skin surrounding the treatment site. Some patients have or develop allergy against salicylic acid. Thus, salicylic acid cannot be used for these patients. In general, salicylic acid should not be used for patients with diabetes, peripheral arterial disease, or other circulatory problems. Further, for salicylic acid, over-the-counter limits are set at 2% for topical left on the face, due to the risk of harming the skin.

Solubility experiments show that salicylic acid is hard to dissolve in ethyl lactate, if the concentration of salicylic acid exceeds 25% w/w, and is precipitated at a concentration of 40% w/w. Surprisingly formic acid has shown to be miscible with ethyl lactate at higher concentrations, providing efficacious compositions for the removal of callus, corns, and/or psoriasis.

There is a continued need for alternative means for the treatment of indications such as callus, corns, and psoriasis. Several demands may be put on such means. It may preferably be inexpensive, easy to manufacture, storage stable, effective, and should work shortly after application, allowing callus, corns, and psoriasis to be eradicated within a short time span. In addition, it should preferably be easy to apply, non-toxic, and not be associated with discomfort for the patient, i.e. it should not have a repulsive smell or be painful for the patient during or after administration.

It has surprisingly been discovered that a composition according to claim 1 has particular advantages for the topical treatment of callus, corns, and/or psoriasis.

The compositions of the present invention provide immediate symptomatic relief of pain. They penetrate and soften keratinous skin immediately. They have proven safe to use without side effects. They allow precise application without harming healthy skin. Further, they allow provision of a product with fits varying sizes of keratinized skin to be treated. There is no need to soak e.g. the feet in water prior or post treatment. There is no need to use a file such as a foot file prior to treatment. Compositions of the invention provide very high efficacy.

In particular, it has surprisingly been discovered that a composition of the invention penetrates hyperkeratinized skin faster than a composition of the prior art. Further, the compositions of the invention tend to stay in the hyperkeratinized layer, without penetrating deeper, thereby avoiding damage to lower layers of skin. This in addition allows compositions of the invention to continue to be efficacious in the hyperkeratinized layer.

It may be speculated that there is a synergistic effect between formic acid and at least one C1-C4 alkyl ester of lactic acid, such as ethyl lactate, providing a strong effect against callus, corns, and/or psoriasis. The alkyl ester, or the mixture of acid and alkyl ester, appears to be able to soften a keratinous layer, allowing the acid to penetrate into the keratinous layer and stay there. Thus, the mixture allows the acid to act throughout the callus, corns, and/or psoriasis, without penetrating beyond the keratinous layer. The mixture appears to be able to penetrate and soften the undesired structure. Further, it appears the present invention allows applying a weaker acid, carrying fewer unwanted side effects while providing penetration to the desired depth. A mixture of formic acid and e.g. ethyl lactate allow a high percentage of acid without precipitation as compared to a mixture of salicylic acid and ethyl lactate.

Without being bound by any theory, the inventor of the present invention speculate that formic acid in itself acts as an active ingredient, while e.g. ethyl lactate acts as a carrier, allowing the concentration of acid to be kept locally sufficiently low to impede irritation for the patient. It is additionally speculated that such a carrier allow the acid to penetrate to the desired depth. Further, the inventor speculates that in this case formic acid and ethyl lactate in the presence of water may react to form lactic acid, which independently acts as an active ingredient. Water may be present in the composition, e.g. as humidity and/or as moisture originating from the patient. Aspects and embodiments of the present invention are provided here. It will be clear for the person skilled in the art that these may be combined.

According to an aspect, the invention concerns the use of formic acid and at least one C1-C4 alkyl ester of lactic acid, preferably ethyl lactate, for the manufacture of a composition for the topical treatment of an indication selected among the group consisting of callus, corns, and psoriasis.

Preliminary experiments indicate that the inclusion in the composition of at least one C1-C4 alkyl ester of lactic acid, preferably ethyl lactate, reduces the contact angle of the composition, i.e. reduces the surface tension. Without being bound by any theory, it may be speculated that the number or strengths of hydrogen bonds in the composition is decreased by the inclusion of one of the alkyl esters, such as ethyl lactate, thereby decreasing the contact angle. Inclusion of water and/or alcohol may increase the contact angle. Decreased contact angle makes it easier for the composition to adhere to callus, corns, and/or psoriasis, and following to be absorbed by callus, corns, and/or psoriasis, i.e. improving the penetration of the composition of the invention to the desired depth. This penetration may further emphasize any antiviral effects of formic acid. Additionally, reduced surface tension makes it easier to apply the composition in the adequate amount, with reduced risk of inadvertently smearing composition on adjacent, healthy skin. The reduced surface tension may easily be tested by placing a drop of the composition of the invention on a plate, and comparing with a drop of a prior art composition, e.g. a composition comprising water and formic acid.

According to an aspect, the invention concerns the use of a composition according to claim 1 for the topical treatment of an indication selected among the group consisting of callus, corns, and psoriasis.

The composition may be administered as a paste, an ointment, a plaster or a pen comprising the composition. An antioxidant may improve the storage stability of the product. Suitable antioxidants comprise Vitamin C.

According to yet an aspect, the invention concerns a pen, comprising a composition according to the invention, for topical administration of a composition according to the invention. A pen has shown to be particularly useful for the application of a pharmaceutical composition according to the invention, as exact control of the precise location of application of the composition may be achieved by the topical application on callus, corns, and/or psoriasis with the pen. This allows avoiding problems of damaging skin around the callus, corns, and/or psoriasis. Further, the composition may be rubbed or massaged into callus, corns, and/or psoriasis with the tip of the pen.

According to an aspect, the invention concerns an adhesive plaster comprising a pharmaceutical composition of the invention. A plaster may maintain the moisture levels, it may ensure the composition is kept on the intended site, and/or it may relieve the treated site of pressure, which is particularly important for callus and corns. Additional advantages may comprise avoiding external stimuli, such as clothes and shoes, rub of the composition.

According to an aspect, the invention concerns a plaster, wherein a doughnut shaped patch surrounds a pharmaceutical composition of the invention.

According to an aspect, the invention concerns a plaster of the invention, which is a hydrocolloid plaster. A hydrocolloid plaster may provide moisture.

According to yet an aspect, the invention concerns a kit of parts comprising a plaster and a pharmaceutical composition of the invention.

According to an aspect, the invention concerns a kit of parts comprising a hydrocolloid plaster and a pen comprising a pharmaceutical composition of the invention, and optionally instructions for the use of said hydrocolloid plaster and pen. The combination of using a pen for application and covering the site of application with a hydrocolloid plaster has shown to be particularly beneficial.

### Detailed Disclosure

According to an aspect, the invention concerns a composition according to claim 1 for the treatment of callus, corns, and psoriasis.

Ethyl lactate, also known as lactic acid ethyl ester, is found naturally, and carries a pleasant odor. Ethyl lactate may be produced from biological sources, e.g. by fermentation. Ethyl lactate may be either the levo (S) or dextro (R) form or a mixture of the two. Industrially produced ethyl lactate may consist of a racemic mixture of levo and dextro forms.

According to an embodiment, ethyl lactate used in the present invention is a racemic mixture.

According to another embodiment, ethyl lactate used in the present invention is ethyl (-)-L-lactate, or more than 50% of the ethyl lactate is ethyl (-)-L-lactate. This may be obtained by using ethyl lactate derived from natural sources. According to a preferred embodiment ethyl lactate is ethylS(-)-2-hydroxy propanoate. Preferably it is obtained by fermentation from sugar.

Compositions according to the present invention preferably comprise ethyl lactate. According to an embodiment it is contemplated that this ingredient may be partly or fully substituted with at least one C1-C4 alkyl ester of lactic acid, or a mixture of any of these. The inventor of the present invention speculates that these alkyl esters may have suitable transport enhancing properties. Among the C1-C4 of relevance for the present invention may be mentioned methyl, ethyl, n-propyl, isopropyl, n-butyl, sec-butyl, isobutyl and tert-butyl esters. If suitable, any or all carboxy groups may be esterified. Monoalkyl, dialkyl and/or trialkyl esters may be suitable. Preferred esters are ethyl esters, further preferred are isopropyl esters.

According to an embodiment it is further contemplated that ethyl lactate may be partly substituted with other ingredients with similar properties, such as lactic acid, preferably L-lactate, malic acid, tartaric acid, citric acid, acetic acid, glycolic acid, propionic acid, 3-hydroxypropanoic acid, malonic acid, butyric acid, hydroxybutyric acid, 1-propanol, 2-propanol, propionaldehyde, acrolein or sodium lactate, or a mixture of any of these.

According to an embodiment, formic acid may be substituted partly by salicylic acid, and/or other ingredients with similar properties, such as lactic acid, preferably L-lactate, malic acid, tartaric acid, citric acid, acetic acid, glycolic acid, propionic acid, 3-hydroxypropanoic acid, malonic acid, butyric acid, hydroxybutyric acid, 1-propanol, 2-propanol, propionaldehyde, acrolein or sodium lactate, or a mixture of any of these.

According to an embodiment, the invention concerns a composition for application with a pen or pad.

According to an embodiment, the invention concerns a composition, wherein the total amount (w/w) of formic acid in the composition is 30-90% more preferred 30-80%, preferably 40-75%, more preferred 50-70%, preferably 55-65%, more preferred about 60%. It appears that using a lower concentration than 85%, such as the use of a 60% or a 30% concentration, alleviates problems with etching of skin surrounding callus, corns, and/or psoriasis to be removed. However, the larger concentrations tend to expedite the removal. Applying a 60% concentration may appear to shorten the time needed for removal to about half the time as compared to a 30% concentration.

According to an embodiment, the invention concerns such a composition, for use on the foot.

According to an embodiment, the invention concerns a composition, wherein the total amount (w/w) of alkyl ester, such as ethyl lactate, in the composition is 10-70% more preferred 20-70%, preferably 25-60%, more preferably 30-50%, more preferred 32-45%, preferably 35-40%.

According to an embodiment, the invention concerns a composition, wherein the total amount (w/w) of said C1-C4 alkyl ester, such as ethyl lactate, in the composition is 40-90%, more preferred 50-85%, preferably 60-80%, more preferred 65-70%.

According to an embodiment, the invention concerns a composition, wherein the composition comprises lactic acid, preferably wherein the total amount (w/w) of lactic acid in the composition is 2-90%, more preferred 3-85%, preferably 5-80%, more preferred 10-70%, preferably 15-65%, more preferably 20-60%, more preferred 30-50%, preferably 35-40%.

According to an embodiment, a composition of the invention may comprise a local anesthetic, such as lidocain.

According to an embodiment, the invention concerns a composition, wherein the total combined amount of formic acid and C1-C4 alkyl ester, such as ethyl lactate, in the composition is at least 60%, more preferred at least 70%, preferably at least 80%, more preferred at least 90%, preferably at least 95%, more preferably at least 97%, more preferred at least 98%, preferably at least 99%, more preferred 100% (w/w).

According to an embodiment, the invention concerns a composition, comprising an ingredient selected among the group consisting of oil, glycerol, petroleum jelly, cocoa butter, and vitamin E. According to additional embodiment, the amount of oil is selected among 1-40%, preferably 2-35%, more preferred 3-30%, preferably 4-25%, more preferred 5-20%, preferably 10-15%. Oil may provide moisture and softness.

According to additional embodiment, the amount of glycerol is selected among 1-40%, preferably 2-35%, more preferred 3-30%, preferably 4-25%, more preferred 5-20%, preferably 10-15%. Glycerol may provide moisture and softness.

According to additional embodiment, the amount of petroleum jelly is selected among 1-40%, preferably 2-35%, more preferred 3-30%, preferably 4-25%, more preferred 5-20%, preferably 10-15%.

According to additional embodiment, the amount of cocoa butter is selected among 1-40%, preferably 2-35%, more preferred 3-30%, preferably 4-25%, more preferred 5-20%, preferably 10-15%.

According to additional embodiment, the amount of vitamin E is selected among 0.1-15%, preferably 0.2-10%, more preferred 0.3-5%, preferably 0.5-4%, more preferred 0.75-3%, preferably 1-2%.

According to a further embodiment of the present invention, pharmaceutically acceptable carriers, such as water, oil, glycerol, alcohol, or mixtures thereof, can be included in a composition according to the invention, e.g. to achieve a further softening effect on and around the callus, corns, moles, telangiectasias, and/or psoriasis.

According to an embodiment, a composition according to the invention may comprise additional active ingredients or excipients. Examples comprise, but are not limited to, Callitris Intratropica, Lavandula Angustifolia, Melaleuca Alternifolia, lemon oil, and mixtures of any of these. The person skilled in the art is familiar with additional pharmaceutically acceptable excipients which may be combined with the present invention.

According to an embodiment, a composition according to the invention may be made substantially free of water and/or ethanol.

According to an embodiment, the invention concerns a composition for the topical treatment of callus, corns, and/or psoriasis comprising formic acid as an active ingredient, and less than 15%, preferably less than 10%, more preferred less than 5%, preferably less than 3%, more preferred substantially no water and/or alcohol such as ethanol.

According to an embodiment, the invention concerns a composition for the topical treatment of callus, corns, and/or psoriasis comprising formic acid as an active ingredient, such as a composition according to the invention, and further comprising at least one colorant, e.g. a dye or a pigment, such as carotene. This embodiment may facilitate correct application on callus, corns, and/or psoriasis, and avoid application on surrounding tissue and skin. Particularly preferred is a colorant with a discrete color, or a color, which vanishes from the callus, corns, and/or psoriasis after application. This may improve patient compliance.

According to an embodiment, a composition according to the invention may be in the form of an emulsion, cream, paste, ointment, lotion, suspension, gel, spray, and/or together with topical carriers suitable for the treatment.

According to an embodiment, a composition according to the invention is applied daily for 4 days, followed by 4 days without application. This cycle may be repeated if necessary. Alternatively, the composition may be applied every second day for two weeks. After a resting period, this cycle may be repeated if necessary.

According to an embodiment, a composition according to the invention may be for human use or veterinary use.

According to an embodiment, the invention concerns a pharmaceutical composition for medical use.

All proportions and percentages are in weight/weight unless otherwise mentioned.

The accompanying Examples are provided to explain rather than limit the present invention. It will be clear to the person skilled in the art that aspects, embodiments and claims of the present invention may be combined.

### Examples

In general, higher formic acid concentration led to higher efficacy. However, complaints were also increasing due to pain upon application of the compositions with higher formic acid contents.

### Contemplated Compositions of the Invention

The compositions of Table I and II have been made or are contemplated. The compositions may be manufactured by mixing the ingredients at room temperature.

The compositions may be applied on callus, corns, and/or psoriasis e.g. as a drop, with a cotton stick or with a pen or an adhesive plaster comprising the composition, (* illustrative examples)

**Table I**

| Composition No. | Formic acid | Ethyl lactate | Lactic acid | Lavender Oil | Callitris intratropica oil | Melaleuca alternifolia oil | Aqua | Glycerol | **Total** |
|---|---|---|---|---|---|---|---|---|---|
| 1 | 85% | 15% | | | | | | | **100%** |
| 2 | 33% | 56% | 10% | 1% | | | | | **100%** |
| 3 * | 3% | 87% | 10% | | | | | | **100%** |
| 4 * | 10% | 80% | 10% | | | | | | **100%** |
| 5 | 50% | 30% | 20% | | | | | | **100%** |
| 6 | 50% | 15% | 30% | 1% | 1% | 1% | 1% | 1% | **100%** |
| 7 * | 20% | 70% | 10% | | | | | | **100%** |
| 8 | 40% | 35% | 20% | 4% | | | | 1% | **100%** |
| 9 | 35% | 40% | 5% | 10% | 1% | 4% | | 5% | **100%** |
| 10 | 60% | 20% | 5% | | | | 10% | 5% | **100%** |
| 11 * | 20% | 30% | 10% | 15% | | | 10% | 15% | **100%** |
| 12 | 80% | 15% | | 3% | | | | 2% | **100%** |
| 13 * | 75% | 5% | 15% | | 5% | | | | **100%** |
| 14 | 70% | 25% | | 2% | | 2% | | 1% | **100%** |
| 15 | 30% | 25% | 40% | | | | | 5% | **100%** |
| 16 | 45% | 15% | 40% | | | | | | **100%** |
| 17 * | 10% | 90% | | | | | | | **100%** |
| 18 * | 15% | 80% | 5% | | | | | | **100%** |
| 19 | 35% | 50% | 10% | | | | 5% | | **100%** |
| 20 | 30% | 15% | 50% | 1% | 1% | 1% | 1% | 1% | **100%** |
| 21 | 40% | 50% | 10% | | | | | | **100%** |
| 22 * | 25% | 50% | 25% | | | | | | **100%** |
| 23 * | 14% | 70% | 10% | 1% | | | | 5% | **100%** |
| 24 | 35% | 65% | | | | | | | **100%** |

**Table II**

| Composition No. | Formic acid | Ethyl lactate | Glycerol | Vitamin E | Cocoa butter | Lanolin | **Total** |
|---|---|---|---|---|---|---|---|
| 25 * | 3% | 97% | | | | | **100%** |
| 26 * | 5% | 95% | | | | | **100%** |
| 27 * | 6% | 94% | | | | | **100%** |
| 28 * | 7% | 93% | | | | | **100%** |
| 29* | 8% | 92% | | | | | **100%** |
| 30 * | 10% | 90% | | | | | **100%** |
| 31 * | 12% | 88% | | | | | **100%** |
| 32 * | 15% | 85% | | | | | **100%** |
| 33 * | 17% | 83% | | | | | **100%** |
| 34 * | 20% | 80% | | | | | **100%** |
| 35 | 30% | 70% | | | | | **100%** |
| 36 | 40% | 60% | | | | | **100%** |
| 37 | 45% | 55% | | | | | **100%** |
| 38 | 60% | 39% | | 1% | | | **100%** |
| 39 | 60% | 34% | 5% | 1% | | | **100%** |
| 40 | 60% | 34% | 5% | | 1% | | **100%** |
| 41 | 60% | 34% | 5% | | | 1% | **100%** |
| 42 | 60% | 32% | 5% | 1% | 1% | 1% | **100%** |
| 43 | 60% | 34% | 3% | 1% | 1% | 1% | **100%** |
| 44 | 60% | 36% | 1% | 1% | 1% | 1% | **100%** |

As indicated in the table, the composition may comprise ingredients such as e.g. glycerol, cocoa butter, vitamin E and/or lanolin. The composition may comprise an antioxidant, such as 1% antioxidant.

### Measurement of Contact Angle

Measurements of the contact angle of mixtures of a) formic acid and water; and b) formic acid and ethyl lactate were conducted according to the following protocol:
1) Coverslip cleaned with methanol were washed with H₂O and dried with N₂.
2) The liquid mixtures were mixed on a rotation table for 10 min.
3) Using 2 coverslips for each comparison, three droplets of each of the samples are added onto each of the two coverslips.

Each solution (mixture) was measured on two distinct surfaces (3 drops on each surface). From the contact angle studies a significant difference between the samples was observed. The results are provided in Table III below.

**Table III**

| Average Measured Contact Angles of Mixtures | + water to 100% (w/w) | +ethyl lactate to 100% (w/w) |
|---|---|---|
| 35% formic acid | 31 | 16 |
| 85% formic acid | 18 | 10 |

### Measurement of effect

The main symptom of corns is the accumulation of dead cells on the outer skin layer, quite resistant to mechanical and chemical aggressions, due to extensive networks of cross-linked proteins and hydrophobic keratin intermediate filaments containing many disulphide bonds. That is the main reason why the penetration of chemical compounds is a great challenge. The presence of the hydrophobic keratin may explain why decreased contact angle of the composition appear preferable.

The use of in vitro transcutaneous diffusion cells allows the study of the penetration of chemical compounds through the skin layers. The results obtained may be used to study the pharmacokinetics of different formulations and thus allow comparison of vehicles for specific active compounds. This provides data showing not only how fast the active compound is crossing the barrier(s), but also how deeply and in which quantity. The effective penetration of the relevant compound(s) is thus measured.

All this will have a direct effect on the quality of the corn treatment and the formulations may be tested taking into account the characteristics of the corn skin and the optimal drug distribution.

Penetration testing is based on franz cell testing (Permegear). It consists of a donor camera and a receptor camera. A membrane imitating the corn skin is placed between the cameras, and a known amount of formulation is applied to the membrane on the donor side. At the receptor camera, a collector liquid with known volume is introduced, e.g. a buffer solution with physiological pH.

The compounds present in the formulation cross the membrane, the driven force being the difference of concentration over the membrane. Samples are taken from the receptor medium every 2nd hours during 24 hours, and analyzed to determine the drug concentration. In order to have statistically acceptable results each formulation is studied at least six times.

The information obtained may be very broad (including, but not limited to, diffusion coefficients and pharmacokinetics parameters). In this case the main focus is obtaining the amount of accumulated drug at the receptor camera versus time. The curves for different formulations allow comparison of vehicles for drug release into the corn.

Preliminary results indicate the present formulation provide efficient treatment of callus, corns, and psoriasis.

### Formic acid study with Franz cells

The three formulations employed are shown in table IV. An artificial membrane is employed in the study. The main purpose of this product is to treat corns, which is a hyperkeratinisation of the skin. It is thus decided to employ a membrane made of keratin. Membranes are purchased at the department of pharmaceuticals in Technische Universität Braunschweig and are made of human hair (Lusiana, Reichl S. and Müller-Goymann C.C., 2011. Keratin film made of human nail as a nail plate model for studying drug permeation. European Journal of Pharmaceutics and Biopharmaceutics, No. 78).

The study was performed using static Franz cells containing 3 mL phosphate buffered saline (pH 7.4, 0.05 % BSA). During the experiment the receptor chambers are maintained at 37 °C. The study was performed with three replicates of each treatment (3+3+3). Samples were drawn at appropriate time points over a course of 24 hours. The formic acid content in samples was analyzed with an enzymatic assay based on a formate dehydrogenase mediated oxidation of formic acid into carbon dioxide with the concurrent formation of NADH in the presence of NAD+. The amount of formed NADH is stoichiometric with the amount of formic acid present in the sample and can be measured spectrophotometrically at 340 nm.

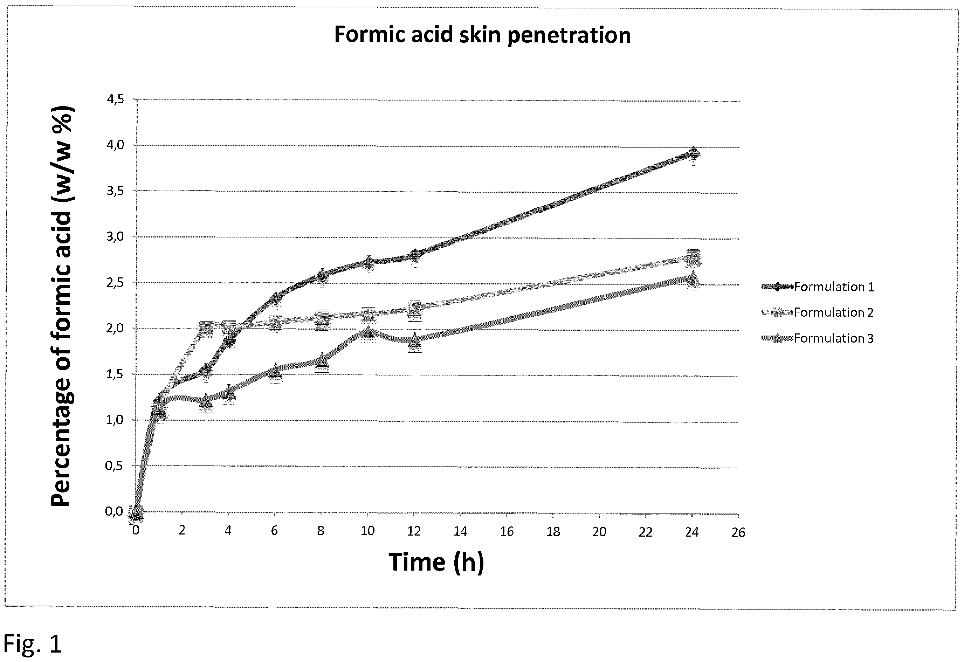

Results obtained are shown in table V and figure 1 (taking into account that at every measurement 200 µL of sample is taken and 200 µL of the medium is added).

As it can be observed, penetration is faster for formulation 2 during the first four hours. Afterwards, formulation 1 penetrates more, reaching a maximum of 4% in 24 hours. The experiment shows that ethyl lactate acts as a penetration enhancer and thus initially this formulation is faster. Further that the presence of ethyl lactate means that the composition does not continue to penetrate through the keratinous layer, thus deeper layers and tissue is left unharmed while the active remains in the keratinous layer.

**Table IV: Formulation employed in the formic acid penetration study**

| **Formulation 1** | **%(w/w)** | **Formulation 2** | **%(w/w)** | **Formulation 3** | **%(w/w)** |
|---|---|---|---|---|---|
| Formic acid | 60 | Formic acid | 60 | Formic acid | 60 |
| Water | 40 | Ethyl lactate | 40 | Ethyl lactate | 25 |
| | | | | Propylene glycol | 15 |

**Table V: Experimental results (after correction)**

| | **Formic acid content in samples from different Franz cells (percent)** | | | | | | | | |
|---|---|---|---|---|---|---|---|---|---|
| | **Formulation 1** | | | **Formulation 2** | | | **Formulation 3** | | |
| **Time (hours)** | **1** | **2** | **3** | **4** | **5** | **6** | **7** | **8** | **9** |
| **0** | 0,003 | 0,002 | 0,002 | 0,002 | 0,002 | 0,002 | 0,002 | 0,002 | 0,002 |
| **1** | 1,241 | 1,213 | 1,189 | 1,093 | 1,198 | 1,045 | 1,074 | 1,079 | 1,243 |
| **2** | 1,455 | 1,434 | 1,533 | 1,436 | 1,400 | 1,647 | 1,362 | 1,450 | 1,356 |
| **3** | 1,436 | 1,547 | 1,653 | 1,483 | 1,546 | 2,991 | 1,513 | 1,106 | 1,043 |
| **4** | 1,393 | 1,783 | 2,450 | 1,595 | 1,590 | 2,888 | 1,263 | 1,421 | 1,275 |
| **6** | 2,231 | 2,110 | 2,657 | 1,850 | 1,681 | 2,681 | 1,562 | 1,732 | 1,360 |
| **8** | 3,096 | 2,298 | 2,364 | 1,840 | 1,737 | 2,803 | 1,572 | 1,639 | 1,794 |
| **10** | 3,114 | 2,469 | 2,605 | 2,022 | 1,979 | 2,501 | 1,872 | 1,877 | 2,183 |
| **12** | 3,001 | 2,579 | 2,865 | 2,339 | 1,961 | 2,392 | 1,865 | 1,680 | 2,127 |
| **24** | 3,514 | 4,256 | 4,044 | 3,053 | 2,820 | 2,527 | 2,317 | 2,765 | 2,672 |
| **donor (24 hr)** | 3,56 | 3,89 | 3,75 | 4,06 | 4,23 | 4,00 | 4,52 | 3,92 | 22,60 |
| **Volume of cell (mL)** | 3,1 | 3,0 | 3.1 | 3,1 | 3,1 | 3,0 | 3,0 | 3,2 | 3,1 |

### Clinical testing

A patient suffered from a corn on the left side of the little toe of the left foot. The patient was treated with a composition comprising 60% formic acid, 39% ethyl lactate and 1% E-vitamin (Composition No. 38) during a period of 20 days, comprising a total of 6 applications. The corn was eradicated completely, leaving no scars. The treatment was not associated with any pain or discomfort for the treated patient.

### Clinical testing II

The safety and efficacy of a composition comprising 60% formic acid and 40% ethyl lactate was tested on 72 cases of corn/calluses. After a two week treatment period 95.8% of the cases had complete or partly resolution. No adverse effects were observed.

### Conclusions

Preliminary experiments indicate selected compositions according to the invention may in favorable cases completely eradicate callus, corns, within 2-3 days upon daily topical administration. Other cases may require longer time, such as 1, 2 or 3 weeks. Compositions of the invention penetrate into hyperkeratinised skin, and stays in the hyperkeratinsed layer, without penetrating further. This allows the composition to continue to be efficacious in the hyperkeratinsed layer. In addition, a composition of the invention showed faster penetration that a prior art composition.

The composition may used against hard corns as well as soft corns. Particular success has been achieved using a composition according to the invention to remove hard corns (heloma durum). The composition is however also applicable to soft corns (heloma molle).

Lower concentrations of formic acid tend to remove the smell and the pain associated with application of a mixture comprising formic acid. The present invention appears to allow the treatment using lower concentrations of formic acid. Lower concentrations allow more frequent, such as daily, application of the composition, and thus the concentration of active ingredients *in situ* may be kept approximately constant as compared to the situation where a composition is applied on a weekly basis. High concentration of formic acid is associated with the disadvantage of pain and creation of wounds upon accidental application of the skin surrounding callus, corns, and/or psoriasis. Thus, high concentration of formic acid usually require longer intervals between application, as the skin needs time to reconstitute between treatments.

A composition of the present invention could readily be applied to the face, arms, legs and body of the patient with good compliance. A composition according to the invention could be applied to other parts of the skin, such as the hands and feet of the patients, with similar good compliance.

## Claims

1. Composition comprising formic acid and at least one C1-C4 alkyl ester of lactic acid or a mixture of any of these, for use in the topical treatment of an indication selected among callus, corns, and psoriasis, wherein the total amount (w/w) of said ester, in the composition is 10 - 70%, and wherein the total amount (w/w) of formic acid in the composition is 30 - 90%.

2. Composition for use according to claim 1, wherein said at least one C1-C4 alkyl ester is ethyl lactate.

3. Composition for use according to any of the preceding claims, wherein the indication is selected among the group consisting of callus and corns.

4. Composition for use according to any of the preceding claims, wherein the total amount (w/w) of formic acid in the composition is 30 - 85%, more preferred 30 - 80%, preferably 40 - 75%, more preferred 50 - 70%, preferably 55 - 65%, more preferred about 60%.

5. Composition for use according to any of the preceding claims, wherein the total amount (w/w) of said C1-C4 alkyl ester, such as ethyl lactate, in the composition is 15 - 65%, more preferably 20 - 60%, more preferred 30 - 50%, preferably 35 - 40%.

6. Composition for use according to any of the preceding claims, wherein the composition further comprises at least one ingredient selected among oil, glycerol, cocoa butter, lanolin, and vitamin E.

7. Composition for use according to claim 1, wherein the composition is in form of cream, lotion or ointment.

8. Pen for topical administration, comprising a composition according to claim 1.

9. Adhesive plaster comprising a composition according to claim 1.

10. Plaster according to claim 9, wherein a doughnut shaped patch surrounds said pharmaceutical composition.

11. Plaster according to claim 9 or 10, wherein said plaster is a hydrocolloid plaster.

12. Kit of parts comprising a plaster and a composition according to claim 1.

13. Kit of parts comprising a hydrocolloid plaster and a pen according to claim 8, and optionally instructions for the use of said hydrocolloid plaster and said pen.

## Patentansprüche

1. Zusammensetzung, umfassend Ameisensäure und mindestens einen C1-C4-Alkylester der Milchsäure oder eine Mischung dieser zur Verwendung bei der topischen Behandlung einer Indikation, ausgewählt aus Hornhaut, Hühneraugen und Psoriasis, wobei die Gesamtmenge (w/w) des Esters in der Zusammensetzung 10 - 70% beträgt und wobei die Gesamtmenge (w/w) der Ameisensäure in der Zusammensetzung 30 - 90% beträgt.

2. Zusammensetzung zur Verwendung nach Anspruch 1, wobei der mindestens eine C1-C4-Alkylester Ethyllactat ist.

3. Zusammensetzung zur Verwendung nach einem der vorhergehenden Ansprüche, wobei die Indikation ausgewählt ist aus der Gruppe bestehend aus Hornhaut und Hühneraugen.

4. Zusammensetzung zur Verwendung nach einem der vorstehenden Ansprüche, wobei die Gesamtmenge (w/w) an Ameisensäure in der Zusammensetzung 30 - 85%, bevorzugter 30 - 80%, vorzugsweise 40 - 75%, bevorzugter 50 - 70%, vorzugsweise 55 - 65%, bevorzugter um 60% beträgt.

5. Zusammensetzung zur Verwendung nach einem der vorstehenden Ansprüche, wobei die Gesamtmenge (w/w) des C1-C4-Alkylesters, wie beispielsweise Ethyllactat, in der Zusammensetzung 15 - 65%, bevorzugter 20 - 60%, bevorzugter 30 - 50%, bevorzugter 35 - 40% ist.

6. Zusammensetzung zur Verwendung nach einem der vorstehenden Ansprüche, wobei die Zusammensetzung ferner mindestens einen Bestandteil umfasst, der ausgewählt ist aus Öl, Glycerin, Kakaobutter, Lanolin und Vitamin E.

7. Zusammensetzung zur Verwendung nach Anspruch 1, wobei die Zusammensetzung in Form von Creme, Lotion oder Salbe vorliegt.

8. Stift zur topischen Verabreichung, umfassend eine Zusammensetzung nach Anspruch 1.

9. Klebepflaster, umfassend eine Zusammensetzung nach Anspruch 1.

10. Pflaster nach Anspruch 9, wobei eine doughnutförmige Stelle die pharmazeutische Zusammensetzung umgibt.

11. Pflaster nach Anspruch 9 oder 10, wobei das Pflaster ein Hydrokolloidpflaster ist.

12. Kit aus Teilen, umfassend ein Pflaster und eine Zusammensetzung nach Anspruch 1.

13. Kit aus Teilen, umfassend ein Hydrokolloidpflaster und einen Stift nach Anspruch 8, und gegebenenfalls eine Anleitung für die Verwendung des Hydrokolloidpflaster und des Stiftes.

## Revendications

1. Composition comprenant de l'acide formique et au moins un ester alkylique en C₁-C₄ de l'acide lactique ou un mélange de l'un quelconque de ces derniers, pour son utilisation dans le traitement par voie locale d'une indication choisie parmi un durillon, de la corne et le psoriasis ; dans laquelle la quantité totale (en masse/masse) dudit ester dans la composition représente de 10 à 70 % ; et dans laquelle la quantité totale (en masse/masse) de l'acide formique dans la composition représente de 30 à 90 %.

2. Composition pour son utilisation selon la revendication 1, dans laquelle ledit au moins un ester alkylique en C₁-C₄ est le lactate d'éthyle.

3. Composition pour son utilisation selon l'une quelconque des revendications précédentes, dans laquelle l'indication est choisie parmi le groupe constitué par un durillon et des cors.

4. Composition pour son utilisation selon l'une quelconque des revendications précédentes, dans laquelle la quantité totale (en masse/masse) de l'acide formique dans la composition représente de 30 à 85 %, de manière plus préférée de 30 à 80 %, de préférence de 40 à 75 %, de manière plus préférée de 50 à 70 %, de préférence de 55 à 65 %, de manière plus préférée s'élève à environ 60 %.

5. Composition pour son utilisation selon l'une quelconque des revendications précédentes, dans laquelle la quantité totale (en masse/masse) dudit ester alkylique en C₁-C₄, tel que le lactate d'éthyle, dans la composition représente de 15 à 65 %, de manière plus préférée de 20 à 60 %, de manière plus préférée de 30 à 50 %, de préférence de 35 à 40 %.

6. Composition pour son utilisation selon l'une quelconque des revendications précédentes, dans laquelle la composition comprend en outre au moins un ingrédient choisi parmi une huile, du glycérol, du beurre de cacao, de la lanoline et la vitamine E.

7. Composition pour son utilisation selon la revendication 1, dans laquelle la composition se présente sous la forme d'une crème, d'une lotion ou d'un onguent.

8. Stylo destiné à une administration par voie locale, comprenant une composition selon la revendication 1.

9. Emplâtre adhésif comprenant une composition selon la revendication 1.

10. Emplâtre selon la revendication 9, dans lequel un timbre possédant une configuration en forme de beignet entoure ladite composition pharmaceutique.

11. Emplâtre selon la revendication 9 ou 10, dans lequel ledit emplâtre est un emplâtre hydrocolloïdal.

12. Kit de composants comprenant un emplâtre et une composition selon la revendication 1.

13. Kit de composants comprenant un emplâtre hydrocolloïdal et un stylo selon la revendication 8, et de manière facultative, un mode d'emploi pour l'utilisation dudit emplâtre hydrocolloïdal et dudit stylo.
